# EUROPEAN PATENT SPECIFICATION

(11) **EP 3 835 766 B1**
(45) Date of publication and mention of the grant of the patent: **01.05.2024**
(21) Application number: 20203000.3
(22) Date of filing: 21.10.2020
(51) Int. Cl.: G01N 23/20, G01N 23/201, G01N 23/202, G01N 23/2055

(54) **DATA ANALYSIS METHOD**
DATENANALYSEVERFAHREN
PROCÉDÉ D'ANALYSE DE DONNÉES

(30) Priority: 11.12.2019 JP 2019223756
(43) Date of publication of application: 16.06.2021
(73) Proprietor: TOYOTA JIDOSHA KABUSHIKI KAISHA, Toyota-shi, Aichi-ken, 471-8571 (JP)
(72) Inventor: YANO, Masao, Aichi-ken, 471-8571 (JP); SHOJI, Tetsuya, Aichi-ken, 471-8571 (JP)
(74) Representative: J A Kemp LLP

(56) References cited:
- WO-A1-2018/025618
- JP-B2- 6 489 529
- US-A- 6 118 850
- US-B2- 7 972 440

## Description

### FIELD

The present disclosure relates to a data analysis method.

### BACKGROUND

Japanese Unexamined Patent Publication No. 2001-116705 discloses, as a conventional method for analyzing a sample, performing quantitative analysis by X-ray diffraction during which using a standardized sample made of the same material as a target of an X-ray source so as to calibrate an intensity of the diffraction X-rays.

### SUMMARY

US 6,118,850, US 7,972,440 B2, WO 2018/025618 A1 and JP 6489529B2 disclose background art.

However, in the above-mentioned conventional method of analysis of a sample, at the stage of analysis of measurement data of the material (sample) being measured by the X-ray diffraction apparatus, there are parts requiring judgment of a human analyzer, so the results of analysis are liable to vary depending on the intuition, experience, etc. of the analyzer. Further, the results of analysis of the analyzer are not evaluated, so precision of analysis is liable to be unable to be secured.

The present disclosure was made focusing on such a problem and has as its object to keep the results of analysis of the measurement data from varying while improving the precision of analysis.

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

To solve above problem,
the data analysis method according to an aspect of the present disclosure is a data analysis method using a data analysis system comprising a processing device, a storage device connected to the processing device, and a communication part connected to the processing device and able to communicate with an external terminal, which data analysis method comprising: a measurement data acquiring step of acquiring measurement data received through the communication part and analyzing a material; a data analysis step of using a learned machine learning model to process the measurement data and output the results of analysis of the measurement data; and a storage processing step of storing in an analysis result database of the storage device a data set including the measurement data and the results of processing obtained by processing the measurement data as an analysis result data set; a learning-use data set acquiring step of acquiring a learning-use data set including results of evaluation of the results of processing of the measurement data received through the communication part and performed at the outside based on the analysis result data set, the results of evaluation including an evaluation score corresponding to the quality of the results of processing obtained from the measurement data; and a learning step of retraining the machine learning model based on the learning-use data set; characterized in that the said evaluation is carried out by a human.

According to this aspect of the present disclosure, a trained machine learning model is used for processing the measurement data, so the results of analysis of the measurement data can be kept from ending up varying according to the intuition, experience, etc. of the analyzer analyzing the measurement data. Further, the machine learning model is retrained based on a learning-use data set including the results of evaluation of the results of processing of the measurement data, so the measurement data is analyzed and the analysis result data set is stored. As a result, the number of points of the learning-use data set increases. Along with this, the machine learning model can be improved in performance and the measurement data can be improved in precision of analysis.

### BRIEF DESCRIPTION OF DRAWINGS

FIG. 1 is a schematic view of the configuration of a material information acquisition system provided with a data analysis system.
FIG. 2 is a view showing one example of an operation sequence of a material information system.

### DESCRIPTION OF EMBODIMENTS

Below, referring to the drawings, embodiments of the present disclosure will be explained in detail. Note that, in the following explanation, similar constituent elements are assigned the same reference notations.

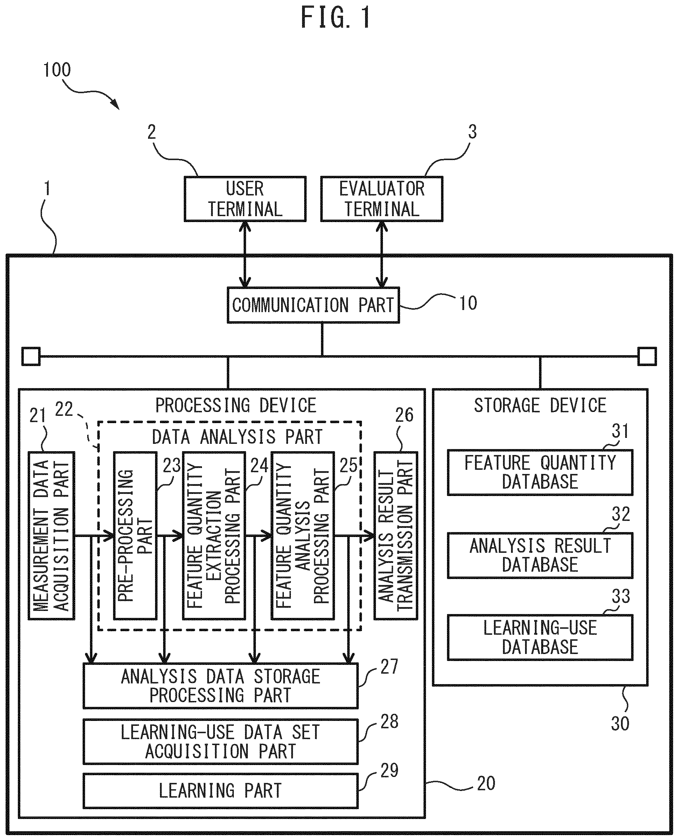

FIG. 1 is a schematic view of the configuration of a material information acquisition system 100 provided with a data analysis system 1 according to one embodiment of the present disclosure.

The material information acquisition system 100 is provided with a data analysis system 1 and a user terminal 2 and evaluator terminal 3 connected through the data analysis system 1 and network and able to communicate with the data analysis system 1.

The material information acquisition system 100 is configured to analyze measurement data (input data) which is input to the data analysis system 1 by one or more users utilizing the material information acquisition system 100 and operating the user terminal 2 and which is measured by measurement devices for material analysis use etc. by the data analysis system 1 using a trained machine learning model and configured to be able to output the results of analysis of the measurement data such as the constituents of the material or chemical state, chemical structure, physical properties, and other related information (below, referred to as the "material information") as output data to the user terminal 2 of the user inputting the measurement data.

Further, the material information acquisition system 100 is configured to retrain the machine learning model used for data analysis based on a later explained learning-use data set which an evaluator evaluating the results of analysis of the measurement data analyzed by the data analysis system 1 inputs to the data analysis system 1 by operating the evaluator terminal 3.

Below, referring to FIG. 1, the hardware configuration of the data analysis system 1, user terminal 2, and evaluator terminal 3 forming the material information acquisition system 100 will be explained.

The user terminal 2 is a device for transfer of information through the network between the data analysis system 1 and the one or more users utilizing the material information acquisition system 100. The user terminal 2, for example, is a computer provided at the user side and provided with a keyboard, display, etc. Note that a user terminal 2 can be provided for each user when there are a plurality of users utilizing the material information acquisition system 100.

The evaluator terminal 3 is a device for transfer of information through the network between the data analysis system 1 and the evaluator evaluating results of analysis of the measurement data analyzed by the data analysis system 1. The evaluator is a human expert specializing in analysis of measurement data. The evaluator terminal 3 is, for example, a computer provided at the evaluator side and having a keyboard, display, etc. Note that, in this embodiment, the person at the supplier side providing the material information acquisition system 100 to the user was specified as the evaluator, but a person at the user side may also be specified as the evaluator.

The data analysis system 1 is provided with a communication part 10, processing device 20, and storage device 30.

The communication part 10 is a communication interface circuit for connecting the data analysis system 1 through a network to the user terminal 2 and the evaluator terminal 3 and enabling communication between the data analysis system 1 and the terminals 2 and 3.

The processing device 20 is a device running various types of programs stored in the storage device 30, for example, a CPU (central processing unit). The processing device 20 performs processing according to the programs to thereby function as a measurement data acquisition part 21, data analysis part 22 (pre-processing part 23, feature quantity extraction processing part 24, and feature quantity analysis processing part 25), analysis result transmission part 26, analysis data storage processing part 27, learning-use data set acquisition part 28, and learning part 29 and operates as a functional part realizing a predetermined function (module). In the following explanation, when explaining the processing using a functional part as the subject, this will indicate that the processing device 20 is running a program for realizing the functional part. Details of the functional parts 21 to 29 will be explained later.

The storage device 30 is a device storing programs which the processing device 20 runs data used when running the programs, for example, a memory, HDD (hard disk drive), SSD (solid state drive), RAM (random access memory), ROM (read only memory), etc. The data stored in the databases of the storage device 30, that is, a feature quantity database 31, analysis result database 32, and learning-use database 33, will be explained in detail later.

Next, referring to FIG. 1, the operation of the material information acquisition system 100 will be explained.

The user can input measurement data of a material acquired at the user side through the user terminal 2 to the data analysis system 1 to thereby obtain results of analysis of the measurement data analyzed by the data analysis system 1, that is, material information, through the user terminal 2 as output. As the measurement data, various types of data obtained by measurement for analysis of the material, for example, data obtained by firing X-rays or neutron beams or electron beams at the material (specifically, measurement data by X-ray diffraction (XRD) or X-ray absorption fine structure analysis (XAFS), X-ray photoemission spectrometry (XPS), X-ray absorption spectroscopy (XAS), X-ray absorption circular dichroism, small angle scattering (SAS), small-angle neutron scattering (SANS), neutral reflectance, inelastic scattering, electron diffraction, etc.), image data of the material observed by a microscope etc. (specifically, image data by an X-ray microscope, optical microscope, electron microscope, atomic force microscope, computer tomography, transmission electron beam imaging, scan electron beam imaging, etc.), etc. can be used.

In this embodiment, as the measurement data, the measurement data when analyzing a material (sample) by an X-ray diffraction apparatus, is input to the user.

If measurement data is input through the user terminal 2 by the user, the data analysis system 1 extracts the feature quantity of the measurement data and analyzes the material based on the extracted feature quantity. In this embodiment, as the feature quantity of the measurement data of the material analyzed by the X-ray diffraction apparatus, the diffraction peak position or intensity, crystal phase, phase fraction, peak width, or other diffraction pattern is extracted and the material is analyzed (phase identified etc.) based on the extracted diffraction pattern.

Below, the content of specific processing performed at the data analysis system 1, that is, the contents of the functional parts 21 to 29 realized by the processing device 20 performing processing in accordance with programs, will be explained.

The measurement data acquisition part 21 acquires measurement data input by the user and outputs it to the data analysis part 22 and analysis data storage processing part 27.

The pre-processing part 23, feature quantity extraction processing part 24, and feature quantity analysis processing part 25 of the data analysis part 22 use respectively trained machine learning models to process the measurement data and output results of analysis comprised of the material information as the final output data. The machine learning models used in the data analysis part 22 are not particularly limited. A neural network, support vector machine, random forest, or various other types of machine learning models can be used.

The pre-processing part 23 receives as input the measurement data acquired by the measurement data acquisition part 21. The pre-processing part 23 pre-processes the input measurement data to, for example, smooth it, remove background, or otherwise reduce noise of the measurement data, that is, pre-processes it to raise the signal-noise ratio, and outputs the measurement data which was pre-processed (below, referred to as the "pre-processed measurement data") to the feature quantity extraction processing part 24 and analysis data storage processing part 27.

The pre-processing part 23 is trained in advance in accordance with the input measurement data to enable suitable smoothing or removal of background. For example, if the pre-processing part 23 smoothes the measurement data by the kernal density estimation method, it has to set the value of the smoothing parameter (band width) for smoothing to for example a suitable value in accordance with the number of data points etc., so the pre-processing part 23 is trained in advance to set a suitable value of the smoothing parameter in accordance with the input measurement data to enable smoothing of the measurement data

The feature quantity extraction processing part 24 receives as input the pre-processed measurement data. The feature quantity extraction processing part 24 is trained in advance so as to enable extraction of a feature quantity of the measurement data in accordance with the input pre-processed measurement data and outputs the extracted feature quantity to the feature quantity analysis processing part 25 and analysis data storage processing part 27. In this embodiment, as explained above, the diffraction peak position or intensity, crystal phase, phase fraction, peak width, or other diffraction pattern is extracted as a feature quantity of the measurement data. Note that the extracted feature quantity is not limited to these. It is also possible to extract for example the distribution of the particle size of the material as the feature quantity in accordance with the measurement data or, if the measurement data is an image, extract a geometric feature in the image as the feature quantity.

The feature quantity analysis processing part 25 receives as input the feature quantity of the measurement data. The feature quantity analysis processing part 25 analyzes the material based on the input feature quantity of the measurement data and outputs the results of analysis to the analysis result transmission part 26 and analysis data storage processing part 27. In this embodiment, the feature quantity analysis processing part 25 compares the diffraction pattern input as the feature quantity of the measurement data with data relating to the feature quantities of known materials stored in the feature quantity database 31, that, the diffraction patterns of known materials, is retrained so as to be able to select a diffraction pattern with a high similarity from the diffraction patterns of known materials, and outputs the material information specified from the selected diffraction pattern as the results of analysis to the analysis result transmission part 26 and analysis data storage processing part 27.

In the past, for example the value of the smoothing parameter was set in accordance with the number of data points by the intuition, experience, etc. of the analyzer. Further, a feature quantity of the measurement data was extracted, the material was analyzed based on the extracted feature quantity, etc. in the same way by intuition, experience, etc. of the analyzer. As opposed to this, in this embodiment, the trained machine learning model was used to process the measurement data. For this reason, the smoothing parameter can be set, the feature quantity of the measurement data can be extracted, and the material can be analyzed based on the feature quantity while keeping them from ending up being dependent on the analyzer like in the past.

The analysis result transmission part 26 sends the input results of analysis, that is, material information, as the results of analysis of the measurement data which the user input to the user terminal 2.

The analysis data storage processing part 27 links the data input to the analysis data storage processing part 27, that is, the measurement data, the pre-processed measurement data obtained by pre-processing that measurement data, the feature quantity extracted from the measurement data, and the results of analysis of the measurement data (material information) and stores them as the analysis result data set in the analysis result database 32.

The evaluator operates the evaluator terminal 3 to access the analysis result database 32 and thereby acquire the analysis result data set, analyzes the relationship between the input data and the output data, and determines an evaluation score corresponding to the quality of the output data obtained from the input data. Furthermore, the evaluator operates the evaluator terminal 3 to input the learning-use data set linking the input data, the output data, and evaluation score to the data analysis system 1.

In this embodiment, the evaluator referred to the measurement data and the pre-processed measurement data obtained by pre-treating that measurement data to, for example, evaluate whether a suitable value was set as the smoothing parameter or otherwise whether the measurement data was suitably pre-processed and assigns an evaluation score corresponding to the results of evaluation to the pre-processed measurement data. At that time, if the measurement data was suitably pre-processed, a high evaluation score is assigned.

Further, the evaluator refers to the measurement data (or pre-processed measurement data) and the feature quantity extracted from the measurement data to, for example, evaluate whether noise hasn't been extracted as the peak or otherwise whether a feature quantity has been suitably extracted from the measurement data and assigns an evaluation score corresponding to the results of evaluation to the feature quantity extracted from the measurement data. At that time, if the feature quantity is suitably extracted from the measurement data, a high evaluation score is assigned.

Furthermore, the evaluator refers to the feature quantity extracted from the measurement data and the results of analysis of the measurement data based on that feature quantity to, for example, evaluate whether a diffraction pattern with a high degree of similarity, whereby the diffraction pattern input as the feature quantity and the diffraction peak position or number of the same match or are similar, has been suitably selected from the feature quantity database 31 or otherwise whether the data was suitably analyzed based on the feature quantity extracted from the measurement data, and assigns an evaluation score corresponding to the results of evaluation to the results of analysis of the measurement data. At this time, if the data as suitably analyzed based on the feature quantity extracted from the measurement data, a high evaluation score is assigned.

Further, the evaluator links the measurement data, the pre-processed measurement data obtained by pre-processing that measurement data, the feature quantity extracted from the measurement data, the results of analysis of the measurement data, the evaluation score assigned to the pre-processed measurement data, the evaluation score assigned for the feature quantity, and the evaluation score assigned for the results of analysis and inputs them as the learning-use data set to the data analysis system 1.

The learning-use data set input to the data analysis system 1 in this way is acquired by the learning-use data set acquisition part 28 and stored in the learning-use database 33 storage device 30. That is, the learning-use data set acquisition part 28 acquires the learning-use data set input by the evaluator and stores the acquired learning-use data set to the learning-use database 33.

The learning part 29 retrains the machine learning model of the data analysis part 22 based on the learning-use data set stored in the learning-use database 33 to optimize the machine learning model. For example, the learning part 29 retrains the machine learning model of the data analysis part 22 when the number of data points of the learning-use data set newly stored in the learning-use database 33 becomes equal to or greater than a predetermined number so as to update the values of the functions used for the machine learning model and optimize the machine learning model.

In this embodiment, the learning part 29 acquires, as the learning-use data set for training the pre-processing part 23, the measurement data, the pre-processed measurement data obtained by pre-processing the measurement data, and the evaluation score assigned to the pre-processed measurement data from the learning-use database 33 and, based on these, optimizes the machine learning model used in the pre-processing part 23 so that the evaluation score of the pre-processed measurement data when the new measurement data is input is maximized. Due to this, for example, in this embodiment, the various types of parameters of the model for calculating the value of the smoothing parameter used at the time of kernal density estimation are optimized.

Further, the learning part 29 acquires, as the learning-use data set for training the feature quantity extraction processing part 24, the measurement data (or pre-processed measurement data), the feature quantity extracted from the measurement data, and the evaluation score assigned for the feature quantity from the learning-use database 33 and, based on these, optimizes the machine learning model used in the feature quantity extraction processing part 24 so that the evaluation score of the feature quantity when new measurement data is input is maximized.

Further, the learning part 29 acquires, as the learning-use data set for training the feature quantity analysis processing part 25, the feature quantity extracted from the measurement data, the results of analysis of the measurement data based on the feature quantity, and the evaluation score assigned for the results of analysis from the learning-use database 33 and, based on these, optimizes the machine learning model used in the feature quantity analysis processing part 25 so that the evaluation score of the results of analysis of the measurement data based on the feature quantity when a new feature quantity is input is maximized.

In this way, in this embodiment, the performances of the machine learning models of the pre-processing part 23, feature quantity extraction processing part 24, and feature quantity analysis processing part 25 are evaluated by the evaluator and the results of evaluation are input to the data analysis system 1. The thus input results of evaluation can be utilized to retrain the pre-processing part 23, feature quantity extraction processing part 24, and feature quantity analysis processing part 25. Due to this, each time the measurement data is analyzed by the data analysis system 1 and the results of evaluation of the results of analysis is utilized to retrain the machine learning model, the machine learning models can be improved in performance.

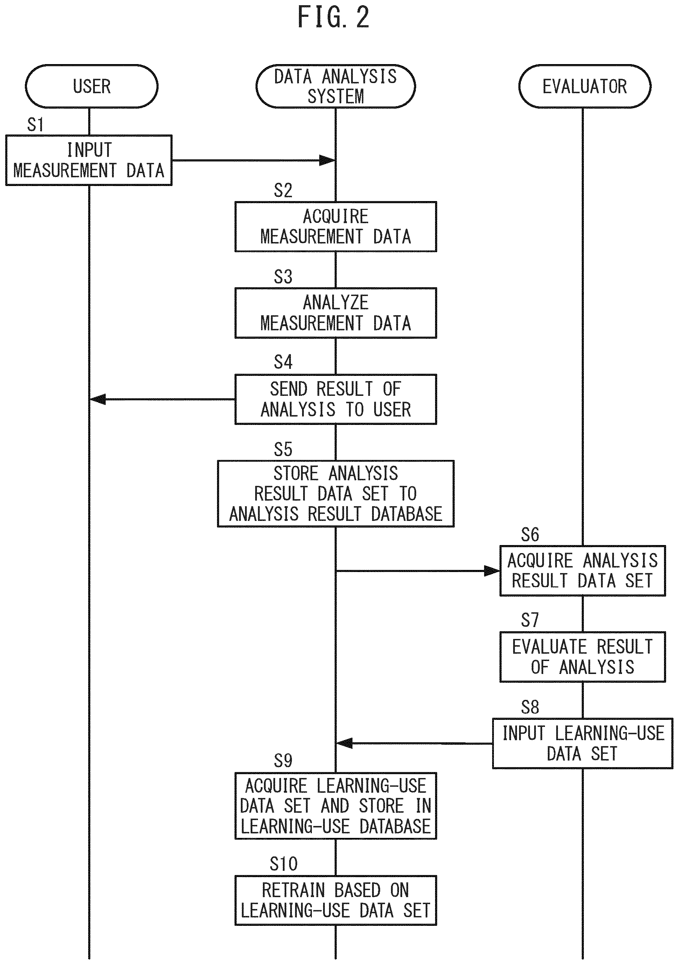

FIG. 2 is a view showing one example of an operation sequence of the material information acquisition system 100.

At step S1, if a user operates the user terminal 2 to input measurement data, the measurement data is sent through the network to the data analysis system 1.

At step S2, the data analysis system 1 acquires the measurement data received through the communication part 10.

At step S3, the data analysis system 1 analyzes the acquired measurement data and outputs the material information as the results of analysis.

At step S4, the data analysis system 1 sends the material information as the results of analysis through the communication part 10 to the user terminal 2 of the user inputting the measurement data.

At step S5, the data analysis system 1 stores the analysis result data set in the analysis result database 32.

At step S6, the evaluator operates the evaluator terminal 3 to acquire the analysis result data set stored in the analysis result database 32 through the network.

At step S7, the evaluator evaluates the results of analysis of the measurement data by the data analysis system 1 based on the analysis result data set and prepares the learning-use data set.

At step S8, if the evaluator operates the evaluator terminal 3 to input the learning-use data set, the learning-use data set is sent through the network to the data analysis system 1.

At step S9, the data analysis system 1 acquires the learning-use data set received through the communication part 10 and stores it in the learning-use database 33.

At step S10, the data analysis system 1 retrains the machine learning model used for analysis of the measurement data based on the learning-use data set stored in the learning-use database 33.

The data analysis system 1 according to the present embodiment explained above is provided with a processing device 20, a storage device 30 connected to the processing device 20, and a communication part 10 connected to the processing device 20 and able to communicate with the external terminals 2, 3. The processing device 20 is provided with a measurement data acquisition part 21 acquiring measurement data received through the communication part 10 and obtained by analyzing a material, a data analysis part 22 using a trained machine learning model to process measurement data and output the results of analysis of the measurement data, an analysis data storage processing part 27 (storage processing part) storing a data set including measurement data and results of processing of processing of the measurement data as an analysis result data set in the storage device 30, a learning-use data set acquisition part 28 acquiring a learning-use data set including results of evaluation of results of processing of the measurement data performed at the outside based on the analysis result data set received through the communication part 10, and a learning part 29 retraining the machine learning model based on the learning-use data set.

In this way, since the data analysis system 1 according to the present embodiment uses a trained machine learning model to process the measurement data, the results of analysis of the measurement data can be kept from ending up varying depending on the intuition etc. and experience etc. of the analyzer analyzing the measurement data. Further, to retrain the machine learning model based on the learning-use data set including the results of evaluation of the results of processing of the measurement data performed at the outside based on the analysis result data set, the measurement data is analyzed and the analysis result data set is stored. As a result, the number of points of the learning-use data set is increased. Along with this, the machine learning model can be improved in performance and the precision of analysis of the measurement data can be improved.

Further, in this embodiment, the processing device 20 is further provided with an analysis result transmission part 26 sending the results of analysis of the measurement data to the outside user terminal 2 from which the measurement data was sent. For this reason, the user can acquire the results of analysis of the measurement data as the output data by just inputting the measurement data.

Further, the data analysis part 22 according to the present embodiment is provided with a feature quantity extraction processing part 24 trained in advance so as to extract and output a feature quantity of the measurement data based on the measurement data and a feature quantity analysis processing part 25 trained in advance so as to output results of analysis of the measurement data corresponding to the feature quantity based on the feature quantity of the measurement data. Further, the data analysis part 22 is further provided with a pre-processing part 23 trained in advance so as to process the measurement data acquired by the measurement data acquisition part 21 to raise the signal-noise ratio and output the pre-processed measurement data and is configured to input the pre-processed measurement data as the input data to the feature quantity extraction processing part 24.

Due to this, it is possible to extract a feature quantity based on the measurement data with the heightened signal-noise ratio, that is, the pre-processed measurement data of the measurement data from which noise has been removed, so it is possible to further improve the precision of analysis of the measurement data.

Further, in this embodiment, the analysis data storage processing part 27 is configured to store in the storage device 30, as an analysis result data set, the measurement data, pre-processed measurement data output from the pre-processing part 23, feature quantity of the measurement data output from the feature quantity extraction processing part 24, and results of analysis of the measurement data output from the feature quantity analysis processing part 25. Further, the learning-use data set includes evaluation scores assigned to output data converted to numerical values corresponding to the quality of the output data output from the pre-processing part 23, feature quantity extraction processing part 24, and feature quantity analysis processing part 25 as results of evaluation of the results of processing of the measurement data. The learning part 29 is configured to retrain the pre-processing part 23 based on the measurement data, pre-processed measurement data output from the pre-processing part 23, and evaluation score assigned to the pre-processed measurement data, retrain the feature quantity extraction processing part 24 based on the measurement data, feature quantity of the measurement data output from the feature quantity extraction processing part 24, and evaluation score assigned for the feature quantity, and retrain the feature quantity analysis processing part 25 based on the measurement data, results of analysis of the measurement data output from the feature quantity analysis processing part 25, and evaluation score imparted to the results of analysis.

Due to this, the pre-processing part 23, feature quantity extraction processing part 24, and feature quantity analysis processing part 25 can be retrained in accordance with the respective results of processing. For this reason, it is possible to improve the performances of the machine learning models of the processing parts 23 to 25 and improve the precision of analysis of the measurement data.

Further, the material information acquisition system 100 according to the present embodiment is provided with a data analysis system 1 and is provided with, as external terminals able to communicate with the data analysis system 1, a user terminal 2 (first terminal) for the user using the data analysis system to input measurement data to the data analysis system 1 and receive the results of analysis of the measurement data as the output data and an evaluator terminal (second terminal) for acquiring an analysis result data set from the storage device 30 and inputting into the data analysis system 1 a learning-use data set including results of evaluation of the results of processing of the measurement data performed based on the acquired analysis result data set.

Due to this, by just inputting the measurement data into the user terminal 2, the user can easily acquire the material information as the results of analysis of the measurement data. Further, in accordance with need, the evaluator can operate the evaluator terminal 3 to acquire the analysis result data set and just input the results of evaluation of the results of analysis as learning-use data set to easily improve the performance in analysis of the data analysis system 1.

Above, embodiments of the present disclosure were explained, but the above embodiments only show some of the examples of application of the present disclosure and are not meant to limit the technical scope of the present disclosure to the specific configurations of the embodiments.

For example, in the above embodiments, the data analysis part 22 was provided with the pre-processing part 23, but if the measurement data does not require pre-processing, the pre-processing part 23 may be omitted. Note that, in this case, the analysis data storage processing part 27 is configured to store in the storage device 30, as an analysis result data set, the measurement data, the feature quantity of the measurement data output from the feature quantity extraction processing part 24, and the results of analysis of the measurement data output from the feature quantity analysis processing part 25. Further, the learning-use data set includes the evaluation score assigned to the output data converted to numerical values according to the quality of the output data output from the feature quantity extraction processing part 24 and feature quantity analysis processing part 25 as the results of evaluation of the results of processing of the measurement data. The learning part 29 is configured for retraining the feature quantity extraction processing part 24 based on the measurement data, the feature quantity of the quantity extraction processing part 24 based on the measurement data, the feature quantity of the measurement data output from the feature quantity extraction processing part 24, and the evaluation score assigned for the feature quantity and for retraining the feature quantity analysis processing part 25 based on the measurement data, the results of analysis of the measurement data output from the feature quantity analysis processing part 25, and the evaluation score assigned for the feature quantity.

In the above embodiments, in accord with the invention, the evaluator was a human expert.

## Claims

1. A data analysis method by a data analysis system (1) comprising a processing device (20), a storage device (30) connected to the processing device (20), and a communication part (10) connected to the processing device (20) and able to communicate with an external terminal, which data analysis method comprising:
a measurement data acquiring step of acquiring measurement data received through the communication part (10) and analyzing a material;
a data analysis step of using a learned machine learning model to process the measurement data and output the results of analysis of the measurement data; and
a storage processing step of storing in an analysis result database of the storage device (30) a data set including the measurement data and the results of processing obtained by processing the measurement data as an analysis result data set;
a learning-use data set acquiring step of acquiring a learning-use data set including results of evaluation of the results of processing of the measurement data received through the communication part (10) and performed at the outside based on the analysis result data set, the results of evaluation including an evaluation score corresponding to the quality of the results of processing obtained from the measurement data; and
a learning step of retraining the machine learning model based on the learning-use data set;
**characterized in that** the said evaluation is carried out by a human.

2. The data analysis method according to claim 1, wherein
the processing device (20) further comprises an analysis result transmission part (26) and the method further comprises the analysis result transmission part (26) sending the results of analysis of the measurement data to the external terminal (2) from which the measurement data was sent.

3. The data analysis method according to claim 1 or claim 2, wherein
the data analysis step is performed by a data analysis part (22) and the data analysis part (22) comprises:
a feature quantity extraction processing part (24) which performs a step of training in advance based on the measurement data so as to extract and output a feature quantity of the measurement data; and
a feature quantity analysis processing part (25) which performs a step of training in advance based on the feature quantity of the measurement data so as to output results of analysis of the measurement data corresponding to the feature quantity.

4. The data analysis method according to claim 3, wherein
the data analysis part (22) further comprises a pre-processing part (23) which performs a step of processing the measurement data acquired in the measurement data acquisition step to raise the signal-noise ratio and trained in advance so as to output the pre-processed measurement data, and
the data analysis part (22) inputs the pre-processed measurement data to the feature quantity extraction processing part (24) as the input data.

5. The data analysis method according to claim 3, further comprising:
storing in the analysis result database, as the analysis result data set, the measurement data, the feature quantity of the measurement data output from the feature quantity extraction processing part (24), and the results of analysis of the measurement data output from the feature quantity analysis processing part (25),
wherein the learning-use data set includes evaluation scores assigned to the output data converted to numerical values corresponding to the quality of the output data output from the feature quantity extraction processing part (24) and the feature quantity analysis processing part (25) as results of evaluation of the results of processing of the measurement data, and
retraining the feature quantity extraction processing part (24) based on the measurement data, the feature quantity of the measurement data output from the feature quantity extraction processing part (24), and the evaluation score assigned for the feature quantity; and
retraining the feature quantity analysis processing part (25) based on the measurement data, the results of analysis of the measurement data output from the feature quantity analysis processing part (25), and the evaluation score assigned to the results of analysis.

6. The data analysis method according to claim 4, further comprising:
storing in the analysis result database, as the analysis result data set, the measurement data, the pre-processed measurement data output from the pre-processing part (23), the feature quantity of the measurement data output from the feature quantity extraction processing part (24), and the results of analysis of the measurement data output from the feature quantity analysis processing part (25),
wherein the learning-use data set includes evaluation scores assigned to the output data converted to numerical values corresponding to the quality of the output data output from the pre-processing part (23), the feature quantity extraction processing part (24), and the feature quantity analysis processing part (25) as results of evaluation of the results of processing of the measurement data, and retraining the pre-processing based on the measurement data, the pre-processed measurement data output from the pre-processing part (23), and the evaluation score assigned to pre-processed measurement data;
retraining the feature quantity extraction processing part (24) based on the measurement data, the feature quantity of the measurement data output from the feature quantity extraction processing part (24), and the evaluation score assigned for the feature quantity; and
retraining the feature quantity analysis processing part (25) based on the measurement data, the results of analysis of the measurement data output from the feature quantity analysis processing part (25), and the evaluation score assigned to the results of analysis.

7. A data analysis method according to any one of claim 1 to claim 6, further comprising:
a user utilizing the data analysis system (1) to input measurement data to the data analysis system (1) and receiving the results of analysis of the measurement data as the output data and
acquiring the analysis result data set from the analysis result database and inputting the learning-use data set including the results of valuation of the results of processing of the measurement data performed based on the acquired analysis result data set to the data analysis system (1).

## Patentansprüche

1. Datenanalyseverfahren durch ein Datenanalysesystem (1), umfassend eine Verarbeitungsvorrichtung (20), eine mit der Verarbeitungsvorrichtung (20) verbundene Speichervorrichtung (30) und einen Kommunikationsteil (10), der mit der Verarbeitungsvorrichtung (20) verbunden ist und in der Lage ist, mit einem externen Endgerät zu kommunizieren,
wobei das Datenanalyseverfahren umfasst:
einen Messdatenerfassungsschritt des Erfassens von Messdaten, die über den Kommunikationsteil (10) empfangen werden, und des Analysierens eines Materials;
einen Datenanalyseschritt des Verwendens eines gelernten maschinellen Lernmodells, um die Messdaten zu verarbeiten und die Ergebnisse der Analyse der Messdaten auszugeben; und
einen Speicherverarbeitungsschritt des Speicherns eines Datensatzes, der die Messdaten beinhaltet, und der Ergebnisse der Verarbeitung, die durch das Verarbeiten der Messdaten erhalten werden, als einen Analyseergebnisdatensatz in einer Analyseergebnisdatenbank der Speichervorrichtung (30);
einen Lern-Verwendungsdatensatzerfassungsschritt des Erfassens eines Lern-Verwendungsdatensatzes, der Ergebnisse der Auswertung der Ergebnisse der Verarbeitung der Messdaten, die durch den Kommunikationsteil (10) empfangen und an der Außenseite auf Grundlage des Analyseergebnisdatensatzes durchgeführt werden, wobei die Ergebnisse der Auswertung eine Auswertungspunktbewertung beinhalten, die der Qualität der Ergebnisse der Verarbeitung entspricht, die aus den Messdaten erhalten werden; und
einen Lernschritt des Neutrainierens des maschinellen Lernmodells auf Grundlage des Lern-Verwendungsdatensatzes;
**dadurch gekennzeichnet, dass** die genannte Auswertung von einem Menschen durchgeführt wird.

2. Datenanalyseverfahren nach Anspruch 1, wobei
die Verarbeitungsvorrichtung (20) ferner einen Analyseergebnisübertragungsteil (26) umfasst und das Verfahren ferner umfasst, dass der Analyseergebnisübertragungsteil (26) die Ergebnisse der Analyse der Messdaten an das externe Endgerät (2) sendet, von dem die Messdaten gesendet wurden.

3. Datenanalyseverfahren nach Anspruch 1 oder 2, wobei
der Datenanalyseschritt von einem Datenanalyseteil (22) durchgeführt wird und der Datenanalyseteil (22) umfasst:
einen Merkmalsquantitätsextraktionsverarbeitungsteil (24), der einen Schritt des Trainierens im Voraus auf Grundlage der Messdaten durchführt, um eine Merkmalsquantität der Messdaten zu extrahieren und auszugeben; und
einen Merkmalsquantitätsanalyseverarbeitungsteil (25), der einen Schritt des Trainierens im Voraus auf Grundlage der Merkmalsquantität der Messdaten durchführt, um Ergebnisse der Analyse der Messdaten, die der Merkmalsquantität entsprechen, auszugeben.

4. Datenanalyseverfahren nach Anspruch 3, wobei
der Datenanalyseteil (22) ferner einen Vorverarbeitungsteil (23) umfasst, der einen Schritt der Verarbeitung der in dem Messdatenerfassungsschritt erfassten Messdaten durchführt, um das Signal-Rausch-Verhältnis zu erhöhen, und im Voraus trainiert wird, um die vorverarbeiteten Messdaten auszugeben, und
der Datenanalyseteil (22) die vorverarbeiteten Messdaten in den Merkmalsquantitätsextraktionsverarbeitungsteil (24) als die Eingabedaten eingibt.

5. Datenanalyseverfahren nach Anspruch 3, das ferner umfasst:
Speichern der Messdaten, der Merkmalsquantität der von dem Merkmalsquantitätsextraktionsverarbeitungsteil (24) ausgegebenen Messdaten und der Ergebnisse der Analyse der von dem Merkmalsquantitätsanalyseverarbeitungsteil (25) ausgegebenen Messdaten in der Analyseergebnisdatenbank als den Analyseergebnisdatensatz,
wobei der Lern-Verwendungsdatensatz Auswertungspunktbewertungen beinhaltet, die den Ausgabedaten zugewiesen sind, die in numerische Werte umgewandelt sind, die der Qualität der Ausgabedaten entsprechen, die von dem Merkmalsquantitätsextraktionsverarbeitungsteil (24) und dem Merkmalsquantitätsanalyseverarbeitungsteil (25) als Ergebnisse der Auswertung der Ergebnisse der Verarbeitung der Messdaten ausgegeben werden, und
Neutrainieren des Merkmalsquantitätsextraktionsverarbeitungsteils (24) auf Grundlage der Messdaten, der Merkmalsquantität der von dem Merkmalsquantitätsextraktionsverarbeitungsteil (24) ausgegebenen Messdaten und der der Merkmalsquantität zugewiesenen Auswertungspunktbewertung; und
Neutrainieren des Merkmalsquantitätsanalyseverarbeitungsteils (25) auf Grundlage der Messdaten, der Analyseergebnisse der Messdaten, die von dem Merkmalsquantitätsanalyseverarbeitungsteil (25) ausgegeben werden, und der den Analyseergebnissen zugewiesenen Auswertungspunktbewertung.

6. Datenanalyseverfahren nach Anspruch 4, das ferner umfasst:
Speichern der Messdaten, der vorverarbeiteten Messdaten, die von dem Vorverarbeitungsteil (23) ausgegeben werden, der Merkmalsquantität der Messdaten, die von dem Merkmalsquantitätsextraktionsverarbeitungsteil (24) ausgegeben werden, und der Analyseergebnisse der Messdaten, die von dem Merkmalsquantitätsanalyseverarbeitungsteil (25) ausgegeben werden, in der Analyseergebnisdatenbank als den Analyseergebnisdatensatz,
wobei der Lern-Verwendungsdatensatz Auswertungspunktbewertungen beinhaltet, die den Ausgabedaten zugewiesen sind, die in numerische Werte umgewandelt werden, die der Qualität der Ausgabedaten entsprechen, die von dem Vorverarbeitungsteil (23), dem Merkmalsquantitätsextraktionsverarbeitungsteil (24) und dem Merkmalsquantitätsanalyseverarbeitungsteil (25) als Ergebnisse der Auswertung der Ergebnisse der Verarbeitung der Messdaten ausgegeben werden, und Neutrainieren der Vorverarbeitung auf Grundlage der Messdaten, der vorverarbeiteten Messdaten, die von dem Vorverarbeitungsteil (23) ausgegeben werden, und der Auswertungspunktbewertung, die den vorverarbeiteten Messdaten zugewiesen ist;
Neutrainieren des Merkmalsquantitätsextraktionsverarbeitungsteils (24) auf Grundlage der Messdaten, der Merkmalsquantität der von dem Merkmalsquantitätsextraktionsverarbeitungsteil (24) ausgegebenen Messdaten und der der Merkmalsquantität zugewiesenen Auswertungspunktbewertung; und
Neutrainieren des Merkmalsquantitätsanalyseverarbeitungsteils (25) auf Grundlage der Messdaten, der Analyseergebnisse der Messdaten, die von dem Merkmalsquantitätsanalyseverarbeitungsteil (25) ausgegeben werden, und der den Analyseergebnissen zugewiesenen Auswertungspunktbewertung.

7. Datenanalyseverfahren nach einem der Ansprüche 1 bis 6, das ferner umfasst, dass:
ein Benutzer das Datenanalysesystem (1) nutzt, um Messdaten in das Datenanalysesystem (1) einzugeben, und die Ergebnisse der Analyse der Messdaten als Ausgabedaten empfängt, und
der Analyseergebnisdatensatz aus der Analyseergebnisdatenbank erfasst wird und der Lern-Verwendungsdatensatz eingegeben wird, der die Ergebnisse der Auswertung der Ergebnisse der Verarbeitung der Messdaten beinhaltet, die auf Grundlage des erfassten Analyseergebnisdatensatzes durchgeführt wurde, in das Datenanalysesystem (1).

## Revendications

1. Procédé d'analyse de données par un système d'analyse de données (1) comprenant un dispositif de traitement (20), un dispositif de stockage (30) connecté au dispositif de traitement (20), et une partie de communication (10) connectée au dispositif de traitement (20) et capable de communiquer avec un terminal externe,
lequel procédé d'analyse de données comprend :
une étape d'acquisition de données de mesure, de l'acquisition de données de mesure reçues par l'intermédiaire de la partie de communication (10), et de l'analyse d'un matériau ;
une étape d'analyse de données, de l'utilisation d'un modèle d'apprentissage machine appris, pour traiter les données de mesure et sortir les résultats d'analyse des données de mesure ; et
une étape de traitement de stockage, du stockage, dans une base de données de résultats d'analyse du dispositif de stockage (30), d'un jeu de données, incluant les données de mesure et les résultats de traitement obtenus en traitant les données de mesure, en tant que jeu de données de résultats d'analyse ;
une étape d'acquisition de jeu de données pour utilisation en apprentissage, de l'acquisition d'un jeu de données pour utilisation en apprentissage, incluant des résultats d'évaluation des résultats de traitement des données de mesure reçues par l'intermédiaire de la partie de communication (10) et réalisé à l'extérieur, sur la base du jeu de données de résultats d'analyse, les résultats d'évaluation incluant une note d'évaluation correspondant à la qualité des résultats de traitement obtenus à partir des données de mesure ; et
une étape d'apprentissage, de la reformation du modèle d'apprentissage machine, sur la base du jeu de données pour utilisation en apprentissage ;
**caractérisé en ce que** ladite évaluation est effectuée par un humain.

2. Procédé d'analyse de données selon la revendication 1, dans lequel
le dispositif de traitement (20) comprend en outre une partie de transmission de résultats d'analyse (26) et le procédé comprend en outre l'envoi, par la partie de transmission de résultats d'analyse (26), des résultats d'analyse des données de mesure, au terminal externe (2) à partir duquel les données de mesure ont été envoyées.

3. Procédé d'analyse de données selon la revendication 1 ou la revendication 2, dans lequel
l'étape d'analyse de données est réalisée par une partie d'analyse de données (22) et la partie d'analyse de données (22) comprend :
une partie de traitement d'extraction de quantité de caractéristique (24) qui réalise une étape de formation d'avance, sur la base des données de mesure, afin d'extraire et de sortir une quantité de caractéristique des données de mesure ; et
une partie de traitement d'analyse de quantité de caractéristique (25) qui réalise une étape de formation d'avance, sur la base de la quantité de caractéristique des données de mesure, afin de sortir des résultats d'analyse des données de mesure correspondant à la quantité de caractéristique.

4. Procédé d'analyse de données selon la revendication 3, dans lequel
la partie d'analyse de données (22) comprend en outre une partie de prétraitement (23) qui réalise une étape de traitement des données de mesure acquises dans l'étape d'acquisition de données de mesure pour élever le rapport signal-bruit et formée d'avance afin de sortir les données de mesure prétraitées, et
la partie d'analyse de données (22) entre les données de mesure prétraitées, dans la partie de traitement d'extraction de quantité de caractéristique (24), en tant que données d'entrée.

5. Procédé d'analyse de données selon la revendication 3, comprenant en outre :
le stockage, dans la base de données de résultats d'analyse, en tant que jeu de données de résultats d'analyse, des données de mesure, de la quantité de caractéristique des données de mesure, sortie depuis la partie de traitement d'extraction de quantité de caractéristique (24), et des résultats d'analyse des données de mesure, sortis depuis la partie de traitement d'analyse de quantité de caractéristique (25),
dans lequel le jeu de données pour utilisation en apprentissage inclut des notes d'évaluation, attribuées aux données de sortie, converties en valeurs numériques correspondant à la qualité des données de sortie, sorties depuis la partie de traitement d'extraction de quantité de caractéristique (24) et la partie de traitement d'analyse de quantité de caractéristique (25), en tant que résultats d'évaluation des résultats de traitement des données de mesure, et
la reformation de la partie de traitement d'extraction de quantité de caractéristique (24), sur la base des données de mesure, de la quantité de caractéristique des données de mesure, sortie depuis la partie de traitement d'extraction de quantité de caractéristique (24), et de la note d'évaluation attribuée pour la quantité de caractéristique ; et
la reformation de la partie de traitement d'analyse de quantité de caractéristique (25), sur la base des données de mesure, des résultats d'analyse des données de mesure, sortis depuis la partie de traitement d'analyse de quantité de caractéristique (25), et des notes d'évaluation attribuées aux résultats d'analyse.

6. Procédé d'analyse de données selon la revendication 4, comprenant en outre :
le stockage, dans la base de données de résultats d'analyse, en tant que jeu de données de résultats d'analyse, des données de mesure, des données de mesure prétraitées sorties depuis la partie de prétraitement (23), de la quantité de caractéristique des données de mesure, sorties depuis la partie de traitement d'extraction de quantité de caractéristique (24), et des résultats d'analyse des données de mesure, sortis depuis la partie de traitement d'analyse de quantité de caractéristique (25),
dans lequel le jeu de données pour utilisation en apprentissage inclut des notes d'évaluation attribuées aux données de sortie, converties en valeurs numériques correspondant à la qualité des données de sortie, sorties depuis la partie de prétraitement (23), la partie de traitement d'extraction de quantité de caractéristique (24), et la partie de traitement d'analyse de quantité de caractéristique (25), en tant que résultats d'évaluation des résultats de traitement des données de mesure, et la reformation du prétraitement, sur la base des données de mesure, des données de mesure prétraitées sorties depuis la partie de prétraitement (23), et des notes d'évaluation attribuées à des données de mesure prétraitées ;
la reformation de la partie de traitement d'extraction de quantité de caractéristique (24), sur la base des données de mesure, de la quantité de caractéristique des données de mesure, sortie depuis la partie de traitement d'extraction de quantité de caractéristique (24), et de la note d'évaluation attribuée pour la quantité de caractéristique ; et
la reformation de la partie de traitement d'analyse de quantité de caractéristique (25), sur la base des données de mesure, des résultats d'analyse des données de mesure, sortis depuis la partie de traitement d'analyse de quantité de caractéristique (25), et des notes d'évaluation attribuées aux résultats d'analyse.

7. Procédé d'analyse de données selon l'une quelconque de la revendication 1 à la revendication 6, comprenant en outre :
par un utilisateur, l'utilisation du système d'analyse de données (1) pour entrer des données de mesure dans le système d'analyse de données (1), et la réception des résultats d'analyse des données de mesure, en tant que données de sortie, et
l'acquisition du jeu de données de résultats d'analyse à partir de la base de données de résultats d'analyse et l'entrée du jeu de données pour utilisation en apprentissage, incluant les résultats de valuation des résultats de traitement des données de mesure, réalisé sur la base du jeu de données de résultats d'analyse acquis, dans le système d'analyse de données (1).
